Europäisches Patentamt

European Patent Office

Office européen des brevets

(11) Publication number: **0 063 494**
**B1**

# EUROPEAN PATENT SPECIFICATION

(45) Date of publication of patent specification: **13.12.89**

(21) Application number: **82302027.6**

(22) Date of filing: **20.04.82**

(51) Int. Cl.⁴: **C 12 N 15/00, C 12 P 21/02, C 12 N 1/20 // C12R1/125**

(54) Method for producing protein from a microorganism, microorganisms for use in such method and creation thereof, vectors for use in said creation, and protein produced thereby, and transformant culture derived from said microorganisms.

(30) Priority: **20.04.81 US 255804**

(43) Date of publication of application:
**27.10.82 Bulletin 82/43**

(45) Publication of the grant of the patent:
**13.12.89 Bulletin 89/50**

(84) Designated Contracting States:
**AT BE CH DE FR GB IT LI LU NL SE**

(56) References cited:
**GB-A-2 039 916**

**JOURNAL OF BACTERIOLOGY, vol. 145, no. 1, Jan 1981; O.GRAY et al.:"Molecular cloning and expression of Bacillus licheniformis beta-lactamase gene in Escherichia coli and Bacillus subtilis", pp. 422-428**

**NATURE, vol. 281, 18 Oct 1979, Macmillan Journals Ltd; D.V.GOEDDEL et al.: "Direct expression in Escherichia coli of a DNA sequence coding for human growth hormone", pp. 544-548**

(73) Proprietor: **CETUS CORPORATION**
**1400 Fifty-Third Street**
**Emeryville California 94608 (US)**

(72) Inventor: **Chang, Shing**
**118 Golden Rod Drive**
**Hercules California 94547 (US)**

(74) Representative: **Bizley, Richard Edward et al**
**BOULT, WADE & TENNANT 27 Furnival Street**
**London EC4A 1PQ (GB)**

(56) References cited:
**NATURE, vol. 287, 02 Oct 1980, Macmillan Journals Ltd.; D.V.GOEDDEL et al.: " Human leucocyte interferon produced by E.coli is biologically active", pp. 411-416**

**PROC. NATL. ACAD. SCI. USA, vol. 77, no. 9, Sep 1980; T.TANIGUCHI et al.: "Expression of the human fibroblast interferon gene in E.coli", pp. 5230-5233**

**SCIENCE, vol. 209, 19 Sep 1980, AAAS; L.GUARENTE et al.: "A technique for expressing eukaryotic genes in bacteria", pp. 1428-1430**

Courier Press, Leamington Spa, England.

## Description

This invention relates to molecular biology and, more particularly, to the so-called art of recombinant DNA. Specifically, the invention relates to a method and cloning vector for the production of cloned heterologous gene products in *Bacillus subtilis* as single unfused proteins.

As is well known, the particular sequence of amino acids in a given protein is determined in accordance with the code carried in the gene for that protein. Proteins are formed from DNA via messenger RNA (mRNA) in the process of translation. During translation, groups of three nucleotides, called codons, in the mRNA direct the placement of one of twenty possible amino acids at a corresponding position in the protein chain.

With the advent of recombinant DNA techniques, genetic changes may be made deliberately by the introduction of a predetermined nucleotide sequence, either synthesized or isolated from one strain or species. The predetermined nucleoride sequence may be selected to cause the strain or species into which it is introduced to produce, as part of the translation process, the protein encoded by the predetermined nucleotide sequence. When the modified strain or species proceeds with the normal replication process, it also then duplicates the inserted sequence.

Recombinant DNA techniques involve isolating a suitable piece of DNA chain (a cloning vector) and breaking or severing the two strands of DNA of the cloning vector at the desired location where the foreign DNA is to be inserted. To do this, particular types of proteins, called restriction enzymes, are typically used. Restriction enzymes will break the DNA at particular nucleotide sequences, although with some restriction enzymes the break may not necessarily occur at the same point on the two intertwined DNA strands. In such a case, if two different types of DNA are severed in a similar manner, the open ends will be complementary and will, under suitable conditions, stick together with the complementary ends lying side by side. They may then be linked together enzymatically with an enzyme such as a ligase. This makes it possible to recombine two DNA segments from any source into a single DNA molecule.

Once the DNA vector has been isolated and the foreign piece inserted therein, the recombinant DNA is then placed into a suitable host organism. In order for the host organism to replicate the inserted DNA, it is necessary that the recombinant DNA be inserted into the host in such a way as to become part of its genetic system. Such insertion can occur in a variety of ways. For example, in the bacterium *Escherichia coli*, two convenient types of cloning vectors have been utilized. *E. coli* bacteria, in addition to the main DNA chain or chromosome, frequently have one or more independently replicating circular loops of DNA known as plasmids. Also a certain type of virus known as lambda bacteriophage (phage) is also capable of infecting *E. coli* and becoming part of its genetic system. Recombinant DNA techniques have included the use of a variety of plasmids or phages as cloning vectors. This involves the isolation of plasmids or phages from the bacteria, the breaking open of the isolated DNA by restriction enzymes, the insertion of a foreign or heterologous piece of DNA into the plasmid or phage so that it is correctly orientated, the restoration of the circular form of the plasmid or the phage structure, and the return of the plasmid or phage to the *E. coli* cell. Once in the host, the heterologous DNA is not only replicated from generation to generation, but also will produce the protein for which it codes if it is correctly oriented and if the proper reading frame and promoters exist.

Most recombinant word to data has been carried out with *E. coli*. Like all bacterial *E. coli* is a prokaryotic cell which has, instead of a true nucleus, a single "naked" chromosome not surrounded by a nuclear membrane. Yeast, on the other hand, are eukaryotes since they contain a true nucleus. Man is also a euykaryote, although one quite distinct from yeast since the two eukaryotes diverged from a common ancestor more than 1.2 billion years ago. Because of the distinct differences between man and yeast, it is not surprising that the two organisms are separated by an evolutionary gap of more than 1.2 billion years. More surprising is the fact that bacterial species can be separated by a similar evolutionary gap. But indeed this is the case for *E. coli* and its distant evolutionary cousin *B. subtilis*. The two claim a common ancestor prior to the time they diverged some 1.2 billion years ago. *See* Hori and Osawa, *Proc. Nat. Acad. Sci.,* USA 76:381—385 (1979).

Nature Vol. 281 (1979) p. 544—548 discloses direct expression in *E. coli* of a heterologous DNA sequence, namely human growth hormone while Nature Vol 287 (1980) p. 411—416 discloses biologically active cloned human leukocyte interferon, again in *E. coli*.

Science Vol 209 (1980) p. 1428—1430 provides teaching as to the minimal requirements to achieve expression in *E. coli* of a cloned eukaryotic gene.

Although certain general approaches to cloning genes in *E. coli* are applicable to other organisms, e.g. yeast and *B. subtilis*, *E. coli* plasmids are not usually capable of replication in *B. subtilis* and *E. coli* genes are not generally expressed in *B. subtilis*. This is sometimes due to differing genetic control mechanisms that result from the great evolutionary gap between the two bacterial species. *See,* Hori and Osawa, *supra.*

*B. subtilis* is preferable to *E. coli* as a host organism because of a greater efficiency for plasmid mediated transformation in *B. subtilis* and because it is non-pathogenic. *B. subtilis* is also a fermentation organism used extensively in industry; therefore its management on an industrial scale is familiar. Although the laboratory *E. coli* strain is a non-pathogenic organism native to the intestines of man and higher animals, there is concern that it might become disease producing if genetically altered. *B. subtilis*, on the other hand, lives in the soil and has never been associated with any disease of plants or animals. Thus there is less likelihood that a genetic alteration in *B. subtilis* would lead to a disease state in man or animals or plants.

In co-pending European Patent Application No. 81300858.8, published 23rd September 1981 with Publication No. 0036259, the first successful production of heterologous gene products by *B. subtilis* is described. This represents a profound advance in the art of genetic engineering. The present invention relates to an advance in the basic technology of the aforesaid Application, in that it demonstrates the first successful production of unfused heterologous protein in *B. subtilis* in which only the desired unfused or pure form of the heterologous protein is produced by the bacteria. The unfused or pure form of the protein product has no extraneous amino acids coded for by sources other than the heterologous gene. The pure heterologous protein is allowed to accumulate within the host. When a desired amount of the pure heterologous protein has accumulated within the host, the pure protein can be recovered by means known to the art.

The invention provides a method for producing a predetermined protein that will accumulate within a transformed host comprising providing growth conditions in a growth media for *B. subtilis* bacteria containing plasmids capable of replication in *B. subtilis* or capable of integrating into the bacterial chromosome of *B. subtilis*, said plasmids having a heterologous gene therein coding for a predetermined protein capable of expression in *B. subtilis*, said heterologous gene including a translational initiation codon sequence and being under the control of operable transcriptional and translational regulatory signals including operator, promoter and ribosomal binding site sequences, said operable transcriptional and translational regulatory signals being indigenous to *B. licheniformis* and there being no translational initiation codon prior to said heterologous gene, and optionally recovering said predetermined protein.

The invention includes a microorganism capable of producing a predetermined protein through expression, comprising a *B. subtilis* host microorganism and a plasmid capable of replication in *B. subtilis* or capable of integrating into the *B. subtilis* bacterial chromosome, said plasmid having a heterologous gene therein coding for a predetermined protein capable of expression in *B. subtilis*, said heterologous gene including a translational initiation codon sequence and being under the control of operable transcriptional and translational regulatory signals including operator, promoter and ribosomal binding site sequences, said operable transcriptional and translational regulatory signals being indigenous to *B. licheniformis* and there being no translational initiation codon prior to said heterologous gene.

Further embraced by the invention is a method for creating a microorganism capable of producing a predetermined protein through expression, comprising forming a plasmid capable of replication in *B. subtilis* or capable of integrating into the *B. subtilis* bacterial chromosome, and having a heterologous gene therein coding for a predetermined protein capable of expression in *B. subtilis*, said heterologous gene including a translational initiation codon sequence and being under the control of operable transcriptional and translational regulatory signals including operator, promoter and ribosomal binding site sequences, said operable transcriptional and translational regulatory signals being indigenous to *B. licheniformis* and there being no translational initiation codon prior to said heterologous gene, and introducing said plasmid to *B. subtilis*.

The invention naturally also provides a plasmid capable of replication in *B. subtilis* or capable of integrating into the *B. subtilis* bacterial chromosome, said plasmid having a heterologous gene therein coding for a predetermined protein capable of expression in *B. subtilis*, said heterologous gene including a translational initiation codon sequence and being under the control of operable transcriptional and translational regulatory signals including operator, promoter and ribosomal binding site sequences, said operable transcriptional and translational regulatory signals being indigenous to *B. licheniformis* and there being no translational initiation codon prior to said heterologous gene.

The invention will now be further described and illustrated by way of the following description; taken in connection with the accompanying drawings wherein:

Figure 1 is a diagram of the human fibroblast interferon gene sequence;

Figure 2 is a diagram of the nucleotide sequence comprising the regulatory region of the *B. licheniformis penP* gene; and

Figure 3 is a diagram of the nucleotide sequence comprising the regulatory sequence fragment from the *B. licheniformis penP* gene used to express the human fibroblast interferon gene.

Very generally, and in accordance with the invention, a predetermined single unfused, protein which is non-indigenous or heterologous to *B. subtilis* is produced through expression by *B. subtilis*. Growth media and conditions are provided for growing a strain of *B. subtilis* in which a plasmid has been introduced. The plasmid is capable of being replicated in the strain or is capable of being integrated into the bacterial chromosome. Such a plasmid carries a properly oriented heterologous gene that codes for the desired predetermined protein. The heterologous gene contains its own translational initiation codon sequence. This translational initiation codon can be naturally present on the heterologous gene or can be placed there synthetically. Appropriate transcriptional and translational regulatory signals, including operator, promoter and ribosomal binding site sequences, from *B. licheniformis*, are also present in the plasmid. The heterologous gene, containing its own translational initiation codon sequence, is located on the plasmid behind a ribosomal binding site sequence at a distance sufficient to provide the spacer nucleotides necessary for proper translation of the heterologous gene. There are no translational initiation codon sequences on the plasmid between the regulator signals and the point at which the heterologous gene is located. Thus, although the heterologous gene is under the control of plasmid regulatory signals, the presence of the translational initiation codon sequence on the heterologous gene insures that the desired initiation of translation will begin at the site of the heterologous gene. As a result, the heterologous gene is expressed as a single unfused peptide. The heterologous gene product will accumulate in the host organism as an intracellular protein. The heterologous gene product can be recovered from within the host organism by means known in the art.

The following examples illustrate ways in which the invention may be employed. They are included only for purposes of illustration and are not intended to limit the scope of the invention in any way.

Example I

Expression of human fibroblast interferon in *B. subtilis* as a single unfused intracellular protein.

The human fibroblast interferon gene for use in the expression of the human fibroblast interferon in *B. subtilis* was isolated by means known to the art. See Taniguchi et al, *Proc. Jpn. Acad.,* Ser. B. 55:464—469 (1979). More specifically, the human fibroblast interferon cDNA clone, 4E1, was obtained by reverse-transcriptase synthesis of cDNA using human mRNA as template and oligo-dT as primer. The cDNA was made double stranded by the action of *E. coli* DNA polymerase I and nicked with S1-nuclease. Homopolymeric tails were added to the 3'-terminal of the double stranded cDNA (dscDNA) by the enzyme terminal-transferase using dCTP as substrate. Similar dG homopolymeric tails were added to the 3'-termini of the plasmid pBR322 which had been linearized at the PstI site. Plasmid pBR322 is on deposit with the American type culture collection. It has been awarded ATOC number 37,017. Samples of the deposit are available to anyone without restriction. The vector and the dscDNA was hybridized and transformed into *E. coli* K12 cells. The clone, 4E1, was identified by Grunstein-Hogness colony hybridization screens using a p[32]-labelled probe and further characterized by restriction enzyme analysis. Such analysis showed that *Pst*I digestion of 4E1 yields two insert fragments of about 600 bp and 200 bp, in addition to a fragment corresponding to linear pBR322. *Bg*lI and *Pst*I digestion of the same clone showed that the 600 *Pst*I insert fragment can be further digested with *Bg*lI to yield two fragments of sizes 358 bp and 200 bp. *Hin*fI digestion of clone 4E1 showed that there are at least three *Hin*fI sites in the insert fragment which generates the three new fragments now present in pBR322.

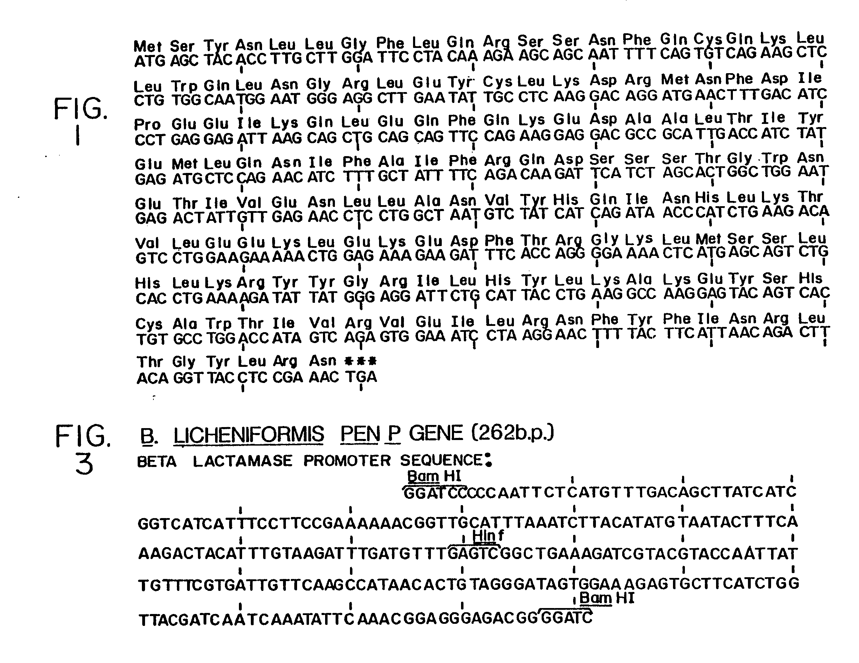

With the aid of restriction mapping data it is possible to obtain the complete nucleotide sequence of the entire human interferon gene. The translated mature protein sequence of the human fibroblast interferon gene is shown in Figure 1.

Prior to its expression in *B. subtilis,* the human fibroblast interferon gene was subcloned for expression in *E. coli*. To accomplish this, the human fibroblast interferon gene coding sequence was subcloned by using a synthetic oligonucleotide primer (TATGAGCTACAAC) and the enzyme DNA polymerase I to degrade DNA sequences 5' to the ATG codon that codes for the amino-terminal methionine of the mature interferon. The repaired DNA was then subcloned into pBR322 at the repaired *Hin*dIII and the *Bam*HI sites. The *Bg*lI site in the human interferon gene, just past the UGA translation termination codon, was used to ligate with the *Bam*HI cohesive end in pBR322 to regenerate an *Xho*II site. The repaired 5'-terminus of the human interferon gene was blunt-end ligated to the repaired *Hin*dIII site; because the original primer has an extra thymidine nucleotide at its 5'-terminus, the *Hin*dIII site was regenerated. The resulting clone pI-25 was confirmed by restriction analysis and DNA sequence analysis.

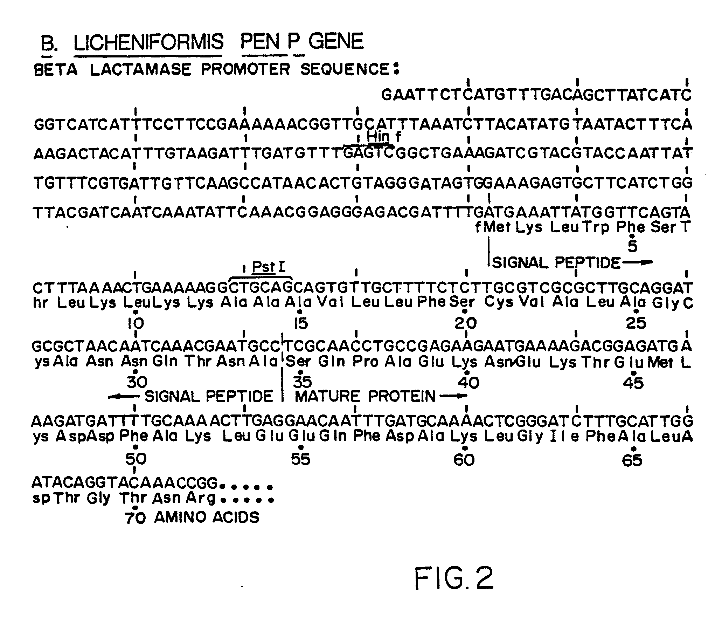

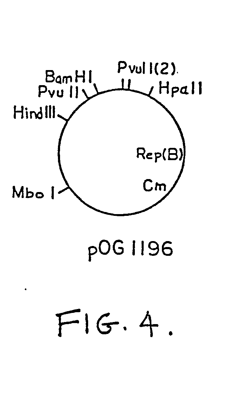

A bacillus penicillinase (beta-lactamase) promoter was used to express the human fibroblast interferon in *B. subtilis*. The bacillus penicillinase (beta-lactamase) promoter fragment was generated from a cloned penicillinase gene. *See.* Gray and Chang, *J. Bacteriol.* 145:422—428 (1981). The coding sequence of the penicillinase (*penP*) gene was digested at the *Pst*I site. Then the linearized DNA was trimmed with BAL-31 exonuclease to remove the coding region just beyond the ATG codon. The coding sequence is outlined in Figure 2. The DNA was then further digested at the *Eco*R1 site located at the 5' end, and repaired by DNA polymerase to generate a blunt-ended fragment. This fragment was further fractionated and purified on acrylamide gel. The purified DNA containing the *penP* gene promoter was then cloned into pLL10 at the *Sma*I site by blunt-end ligation. See Wu, R., (Editor) *Methods in Enzymology,* 68:98—109 (1979). Since the *Sma*I site is flanked by two *Bam*HI sites, it was possible to excise the promoter fragment by *Bam*HI digestion. One plasmid designated as pDH5268, which carries a 262 bp insert, was further characterized by sequencing analysis. Such analysis showed that this plasmid contained the complete promoter sequence as shown in Figure 3.

In order to express the human fibroblast interferon gene in *B. subtilis,* the *E. coli* beta-1—25 plasmid was first converted to a bifunctional replicon by *in vitro* ligation with bacillus plasmid pOG1196 at the *Pvu*II site. The *Bam*HI fragment containing the 262 bp *penP* promoter was then cloned into the *Hin*dIII site 5' to the human fibroblast interferon coding sequence following limited S1 nuclease digestion and *E. coli* DNA polymerase repair. One resulting clone, pDH1151 was further characterized by restriction enzyme analysis. Such analysis showed that in addition to the *Bacillus* derived sequences, this plasmid carried only sequences derived from *E. coli* plasmid pBR322, the nucleotide sequences coding for mature human fibroblast interferon, and the *penP* promoter. Plasmid pDH1151 was then transformed into the *B. subtilis* HVI host strain BGSCIS53. Cell extract prepared from the transformed strain carrying pDH1151 showed that the resulting strain produces intracellular human fibroblast interferon exhibiting antiviral activity comparable to that shown by single unfused mature human fibroblast interferon. *See* Stewart, in *The Interferon System*, Springer-Verlag, New York (1979) at pp. 17—18 for details of antiviral assay.

It may be seen, therefore, that the invention provides a method and a vector for the expression of heterologous cloned genes as single unfused proteins in *B. subtilis*. The unfused heterologous proteins will not contain any extraneous amino acids coded for by DNA originating from either the host or the plasmid. These single unfused protein products will accumulate within the host organism. Following their accumulation within the host organism, the heterologous gene products can be recovered by means known in the art.

Various modifications of the invention in addition to those shown and described herein will become apparent to those skilled in the art from the foregoing description and accompanying drawings. Such modifications are intended to fall within the scope of the appended claims.

Plasmid pOG1196 referred to hereinabove is described in Gray & Chang, S., J. Bacteriol 145:422—428 (1981). Figure 4 gives the map for this plasmid. The plasmid has been deposited with the American type culture collection, Rockville, Maryland 02852, U.S.A. The deposited plasmid has been awarded ATCC number 31,776.

### References

1. Gray, O., and Chang, S., *J. Bacteriol.*145:422—428 (1981).
2. Hori, H., and Osawa, S., *Proc. Nat. Acad. Sci.*, USA 76:381—385 (1979).
3. Stewart, W.E., in *The Interferon System*, Springer-Verlag, New York (1979) at pp. 17 and 18.
4. Taniguchi, T. Sakai, M., Fujii-Kuriyama, Y., Muramatsu, M., Kobayashi, S., and Sudo, T., *Proc. Jpn. Acad.*, Ser. B 55:464—469 (1979).
5. Wu, R., (Editor) *Methods in Enzymology* 68:98—109 (1979).

### Claims

1. A method for producing a predetermined protein that will accumulate within a transformed host comprising providing growth conditions in a growth media for *B. subtilis* bacteria containing plasmids capable of replication in *B. subtilis* or capable of integrating into the bacterial chromosome of *B. subtilis*, said plasmids having a heterologous gene therein coding for the predetermined protein and capable of expression in *B. subtilis*, said heterologous gene including a translational initiation codon sequence and being under the control of operable transcriptional and translational regulatory signals including operator, promoter and ribosomal binding site sequences, said operable transcriptional and translational regulatory signals being indigenous to *B. licheniformis;* and there being no translational initiation codon sequences prior to said heterologous gene, and optionally recovering the resulting predetermined protein.

2. A method as claimed in Claim 1, wherein said heterologous gene for said predetermined protein is under the control of the *B. licheniformis* beta-lactamase transcriptional and translational regulatory signals including operator, promoter and ribosomal binding site sequences.

3. A method as claimed in Claim 1 or Claim 2, wherein said predetermined protein is a eukaryotic protein.

4. A method as claimed in Claim 3, wherein said predetermined protein is a mammalian protein.

5. A method as claimed in Claim 4, wherein said predetermined protein is human fibroblast interferon.

6. A microorganism capable of producing a predetermined protein through expression, comprising a *B. subtilis* host microorganism and a plasmid therein capable of replication in *B. subtilis* or capable of integrating into the *B. subtilis* bacterial chromosome, said plasmid having a heterologous gene therein coding for a predetermined protein capable of expression in *B. subtilis*, said heterologous gene including a translational initiation codon sequence and being under the control of operable transcriptional and translational regulatory signals including operator, promoter and ribosomal binding site sequences, said operable transcriptional and translational regulatory signals being indigenous to *B. licheniformis*, and there being no translational initiation codon prior to said heterologous gene.

7. A method for creating a microorganism capable of producing a predetermined protein through expression, comprising forming a plasmid capable of replication in *B. subtilis* or capable of integrating into the *B. subtilis* bacterial chromosome, and having a heterologous gene including a translational initiation codon sequence and being under the control of operable transcriptional and translational regulatory signals including operator, promoter and ribosomal binding site sequences, said operable transcriptional and translational regulatory signals being indigenous to *B. licheniformis,* and there being no translational initiation codon sequences prior to said heterologous gene, and introducing said plasmid to a *B. subtilis* host microorganism.

8. A plasmid capable of replication in *B. subtilis* or capable of integrating into the *B. subtilis* bacterial chromosome, said plasmid having a heterologous gene therein coding for a predetermined protein capable of expression in *B. subtilis*, said heterologous gene including a translational initiation codon sequence and being under the control of operable transcriptional and translational regulatory signals including operator, promoter and ribosomal binding site sequences, said operable transcriptional and translational regulatory signals being indigenous to *B. licheniformis*, and there being no translational initiation codon prior to said heterologous gene.

9. A microorganism as claimed in Claim 6, or a method as claimed in Claim 7, or a plasmid as claimed in Claim 8, and respectively further defined by the specific feature set out in any one of Claims 2 to 5.

10. A transformant bacterial culture cloned from one or more microorganisms as claimed in Claim 6 or Claim 9, the members of such culture being capable of expressing said predetermined protein.

**Patentansprüche**

1. Verfahren zur Herstellung eines gewünschten, sich in einem transformierten Wirt ansammelnden Proteins, bei dem an Wachstumsbedingungen in einem Wachstumsmedium für B. subtilis-Bakterien schafft, die in B. subtilis replizierbare oder in das B. subtilis-Chromosom integrierbare Plasmide enthalten; wobei die Plasmide ein heterologes, in B. subtilis exprimierbares, das gewünschte Protein codierendes Gen enthalten können; das heterologe Gen eine Translationsinitiationscodon-Sequenz einschließt und unter der Kontrolle von funktionsfähigen Transkriptions- und Translations-Regulationssignalen steht einschließlich Operator, Promoter und Sequenzen für eine robosomale Bindungsstelle; die funktionsfähigen Transkriptions- und Translations-Regulationssignale endogen für B. licheniformis sind; und es vor dem heterologen Gen keine Translationsinitiationscodon-Sequenz gibt; und gegebenenfalls das daraus hervorgehende gewünschte Protein wiedergewinnt.

2. Verfahren nach Anspruch 1, wobei das heterologe Gen für das gewünschte Protein unter der Kontrolle der Transkriptions- und Translations-Regulationssignale einschließlich Operator, Promotor und Sequenzen für eine ribosomale Bindungsstelle der β-Lactamase von B. licheniformis steht.

3. Verfahren nach Anspruch 1 oder 2, wobei das gewünschte Protein ein eukaryontisches Protein ist.

4. Verfahren nach Anspruch 3, wobei das gewünschte Protein ein Säugerprotein ist.

5. Verfahren nach Anspruch 4, wobei das gewünschte Protein ein menschliches Fibroblasten-Interferon ist.

6. Mikroorganismus, der ein gewünschtes Protein durch Expression herstellen kann, umfassend einen B. subtilis-Wertsorganismus und ein Plasmid, wobei das Plasmid in B. subtilis replizierbar oder in das bakterielle B. subtilis-Chromosom integrierbar ist, und wobei das Plasmid ein heterologes, in B. subtilis exprimierbares, das gewünschte Protein codierendes Gen enthalten kann; das heterologe Gen eine Translationsinitiationcodon-Sequenz einschließt und unter der Kontrolle von funktionsfähigen Transkriptions- und Translations-Regulationssignalen steht, einschließlich Operator, Promotor und Sequenzen für eine ribosomale Bindungsstelle; die funktionsfähigen Transkriptions- und Translations-Regulationssignale endogen für B. licheniformis sind; und es vor dem heterologen Gen keine Translationsinitiationscodon-Sequenz gibt.

7. Verfahren zur Herstellung eines Mikroorganismus, der in der Lage ist, ein gewünschtes Protein durch Expression herzustellen, bei dem man ein in B. subtilis replizierbares oder in das bakterielle B. subtilis-Chromoson integrierbares Plasmid erhält; ein heterologes Gen eine Translationsinitiationscodon-Sequenz einschließt und unter der Kontrolle von funktionsfähigen Transkriptions- und Translations-Regulationssignalen einschließlich Operator, Promotor und Sequenzen für eine robosomale Bindungsstelle steht; die funktionsfähigen Transkriptions- und Translations-Regulationssignale endogen für B. licheniformis sind; es vor dem heterologen Gen kein Translationsinitiationscodon-Sequenz gibt; und das Plasmid in einen B. subtilis-Wirtsmikroorganismus einführt.

8. Plasmid, das in B. subtilis replizierbar oder in das bakterielle B. subtilis-Chromosom integrierbar ist, wobei das Plasmid ein für ein gewünschtes Protein codierendes und in B. subtilis exprimierbares heterologes Gen enthält; das heterologe Gen eine Translationsinitiationscodon-Sequenz einschließt; unter der Kontrolle von funktionsfähigen Transkriptions- und Translations-Regulationssignalen einschließlich Operator, Promotor und Sequenzen für eine ribosomale Bindungsstelle steht; die funktionsfähigen Transkriptions- und Translations-Regulationssignale endogen für B. licheniformis sind; und es vor dem heterologen Gen keine Transkriptionsinitiationscodon-Sequenz gibt.

9. Mirkoorganismus nach Anspruch 6 oder Verfahren nach Anspruch 7 oder Plasmid nach Anspruch 8 und entsprechend weiter definiert durch die bestimmten Eigenschaften aus einem der Ansprüche 2 bis 5.

10. Transformierte Bakterienkultur, die aus einem oder mehreren Mikroorganismen nach Anspruch 6 oder Anspruch 9 abgeleitet ist, wobei die Bakterien in der Kultur in der Lage sind, das gewünschte Protein zu exprimieren.

**Revendications**

1. Procédé de production d'une protéine prédéterminée qui s'accumule dans un hôte transformé, dans lequel on établi des conditions de culture dans un milieu de culture de bactéries *B.*

*subtilis* contenant des plamides capables de se répliquer dans *B. subtilis*, ou capables de s'intégrer dans le chromosome bactérien de *B. subtilis*, ces plasmides comportant un gène hétérologue codant pour la protéine prédéterminée, et capable d'être exprimé dans *B. subtilis*, ce gène hétérologue comprenant une séquence de codon d'initiation de traduction, et étant régulé par des signaux de régulation de transcription et de traduction en état de fonctionner, comprenant des séquences d'opérateur, de promoteur et de site de liaison ribosomale, ces signaux de régulation de transcription et de traduction en état de fonctionner, étant originaires de *B. licheniformis*, aucune séquence de codon d'initiation de traduction, n'étant présente avant ledit gène hétérologue; et on récupère éventuellement la protéine prédéterminée obtenue.

2. Procédé selon la revendication 1, dans lequel le gène hétérologue codant pour ladite protéine prédéterminée, est régulé par les signaux de régulation de transcription et de traduction de la béta-lactamase de *B. licheniformis,* comprenant des séquences d'opérateur, de promoteur et de site de liaison ribosomale.

3. Procédé selon la revendication 1 ou la revendication 2, dans lequel la protéine prédéterminée, est une protéine d'eucaryote.

4. Procédé selon la revendication 3, dans lequel la protéine prédéterminée, est une protéine de mammifère.

5. Procédé selon la revendication 4, dans lequel la protéine prédéterminée, est l'interféron de fibroblaste humain.

6. Micro-organisme capable de produire, par expression, une protéine prédéterminée, comprenant un micro-organisme-hôte de l'espèce *B. subtilis*, et un plasmide capable de se répliquer dans *B. subtilis*, ou capable de s'intégrer dans le chromosome bactérien de *B. subtilis*, ce plasmide comportant un gène hétérologue, codant pour une protéine prédéterminée, capable d'être exprimé dans *B. subtilis*, ce gène hétérologue comprenant une séquence de codon d'initiation de traduction, et étant régulé par des signaux de régulation de transcription et de traduction en état de fonctionner, comprenant des séquences d'opérateur, de promoteur et de site de liaison ribosomale, ces signaux de régulation de transcription et de traduction en état de fonctionner, étant originaires de *B. licheniformis*, et aucun codon d'initiation de traduction, n'étant présent avant ledit gène hétérologue.

7. Procédé de création d'un micro-organisme capable de produire, par expression, une protéine prédéterminée, dans lequel on forme un plasmide capable de se répliquer dans *B. subtilis*, ou capable de s'intégrer dans le chromosome bactérien de *B. subtilis*, et comportant un gène hétérologue comprenant une séquence de codon d'initiation de traduction, et régulé par des signaux de régulation de transcription et de traduction en état de fonctionner, comprenant des séquences d'opérateur, de promoteur et de site de liaison ribosomale, ces signaux de régulation de transcription de traduction en état de fonctionner, étant originaires de *B. licheniformis*, aucune séquence de codon d'initiation de traduction, n'étant présente avant ledit gène hétérologue; et on introduit ce plasmide dans un micro-organisme-hôte de l'espèce *B. subtilis*.

8. Plasmide capable de se répliquer dans *B. subtilis*, ou capable de s'intégrer dans le chromosome bactérien de *B. subtilis*, ce plasmide comportant un gène hétérologue codant pour une protéine prédéterminée, capable d'être exprimé dans *B. subtilis*, ce gène hétérologue comprenant une séquence de codon d'initiation de traduction, et étant régulé par des signaux de régulation de transcription de traduction en état de fonctionner, comprenant des séquences d'opérateur, de promoteur et de site de liaison ribosomale, ces signaux de régulation de transcription de traduction en état de fonctionner, étant originaires de *B. licheniformis,* et aucun codon d'initiation de traduction, n'étant présent avant ledit gène hétérologue.

9. Micro-organisme selon la revendication 6, procédé selon la revendication 7, plasmide selon la revendication 8, respectivement définis par les caractéristiques spécifiques mentionnées dans l'une quelconque des revendications 2 à 5.

10. Culture de bactéries transformées, clonées à partir d'un ou plusieurs micro-organismes selon la revendication 6 ou la revendication 9, les organismes de cette culture étant capables de produire par expression, ladite protéine prédéterminée.

EP 0 063 494 B1

FIG.
1

```
Met Ser Tyr Asn Leu Leu Gly Phe Leu Gln Arg Ser Ser Asn Phe Gln Cys Gln Lys Leu
ATG AGC TAC ACC TTG CTT GGA TTC CTA CAA AGA AGC AGC AAT TTT CAG TGT CAG AAG CTC

Leu Trp Gln Leu Asn Gly Arg Leu Glu Tyr Cys Leu Lys Asp Arg Met Asn Phe Asp Ile
CTG TGG CAA TGG AAT GGG AGG CTT GAA TAT TGC CTC AAG GAC AGG ATG AAC TTG GAC ATC

Pro Glu Glu Ile Lys Gln Leu Glu Gln Phe Gln Lys Glu Asp Ala Ala Leu Thr Ile Tyr
CCT GAG GAG ATT AAG CAG CTG CAG CAG TTC CAG AAG GAG GAC GCC GCA TTG ACC ATC TAT

Glu Met Leu Gln Asn Ile Phe Ala Ile Phe Arg Gln Asp Ser Ser Ser Thr Gly Trp Asn
GAG ATG CTC CAG AAC ATC TTT GCT ATT TTC AGA CAA GAT TCA TCT AGC ACT GGC TGG AAT

Glu Thr Ile Val Glu Asn Leu Leu Ala Asn Val Tyr His Gln Ile Asn His Leu Lys Thr
GAG ACT ATT GTT GAG AAC CTC CTG GCT AAT GTC TAT CAT CAG ATA ACC CAT CTG AAG ACA

Val Leu Glu Glu Lys Leu Glu Lys Glu Asp Phe Thr Arg Gly Lys Leu Met Ser Ser Leu
GTC CTG GAA GAA AAA CTG GAG AAA GAA GAT TTC ACC AGG GGA AAA CTC ATG AGC AGT CTG

His Leu Lys Arg Tyr Tyr Gly Arg Ile Leu His Tyr Leu Lys Ala Lys Glu Tyr Ser His
CAC CTG AAA AGA TAT TAT GGG AGG ATT CTG CAT TAC CTG AAG GCC AAG GAG TAC AGT CAC

Cys Ala Trp Thr Ile Val Arg Val Glu Ile Leu Arg Asn Phe Tyr Phe Ile Asn Arg Leu
TGT GCC TGG ACC ATA GTC AGA GTG GAA ATC CTA AGG AAC TTT TAC TTC ATT AAC AGA CTT

Thr Gly Tyr Leu Arg Asn * * *
ACA GGT TAC CTC CGA AAC TGA
```

FIG.
3

## B. LICHENIFORMIS PEN P GENE (262b.p.)

BETA LACTAMASE PROMOTER SEQUENCE:

Bam HI
GGATCCCCC AATT CT CATG TT TGAC AGCT TATC ATC

GGT CATCATTTCCTTCCGAAAAAAC GGTTGCATTTAAATCTTACATATG TAATACTTTCA

Hin f
AAGACTACAT TTGTAAGAT TTGATGT TTGAGTCGGC TGAAAGATCGTACGTACCA ATTAT

TGTTTCGTGATTGTTCAAGCCATAAC ACTG TAGGGATAGTGGAA AGAGTGCTTCATCTGG

Bam HI
TTACGATCA ATC AAATATTC AAAC GGA GGGAGAC GG GGATC

## B. LICHENIFORMIS PEN P GENE

BETA LACTAMASE PROMOTER SEQUENCE:

```
                                    GAATTCTCATGTTTGACAGCTTATCATC

GGTCATCATTTCCTTCCGAAAAAACGGTTGCATTTAAATCTTACATATGTAATACTTTCA
                              Hin f
AAGACTACATTTGTAAGATTTGATGTTTGAGTCGGCTGAAAGATCGTACGTACCAATTAT

TGTTTCGTGATTGTTCAAGCCATAACACTGTAGGGATAGTGGAAAGAGTGCTTCATCTGG

TTACGATCAATCAAATATTCAAACGGAGGGAGACGATTTTGATGAAATTATGGTTCAGTA
                                            fMet Lys LeuTrp Phe Ser T
                                                              5
                                            |SIGNAL PEPTIDE→

              Pst I
CTTTAAAACTGAAAAAGGCTGCAGCAGTGTTGCTTTTCTCTTGCGTCGCGCTTGCAGGAT
hr Leu Lys LeuLys Lys Ala Ala Ala Val Leu Leu Phe Ser Cys Val Ala Leu Ala Gly C
     10              15              20              25

GCGCTAACAATCAAACGAATGCCTCGCAACCTGCCGAGAAGAATGAAAAGACGGAGATGA
ys Ala Asn Asn Gln Thr Asn Ala Ser Gln Pro Ala Glu Lys AsnGlu Lys Thr Glu Met L
     30              35      40              45
     ←SIGNAL PEPTIDE | MATURE PROTEIN→

AAGATGATTTTGCAAAACTTGAGGAACAATTTGATGCAAAACTCGGGATCTTTGCATTGG
ys AspAsp Phe Ala Lys Leu Glu Glu Gln Phe Asp Ala Lys Leu Gly Ile PheAla LeuA
     50          55          60          65

ATACAGGTACAAACCGG.....
sp Thr Gly Thr Asn Arg.....
    70 AMINO ACIDS
```

## FIG.2

pOG 1196

FIG. 4.